# EUROPEAN PATENT SPECIFICATION

(11) **EP 0 677 530 B1**
(45) Date of publication and mention of the grant of the patent: **04.06.1997**
(21) Application number: 95105285.1
(22) Date of filing: 07.04.1995
(51) Int. Cl.: C07H 17/08

(54) **Process for the preparation of azithromycin dihydrochloride**
Verfahren zur Herstellung von azithromycin Dihydrochloriden
Procédé pour la préparation d'azithromycin dihydrochloride

(30) Priority: 15.04.1994 HR 94025194
(43) Date of publication of application: 18.10.1995
(73) Proprietor: PLIVA, farmaceutska, kemijska, prehrambena i kozmeticka industrija dionicko drustvo, 41000 Zagreb (HR)
(72) Inventor: Lopotar, Nevenka, HR-41000 Zagreb (HR); Mutak, Stjepen, HR-41000 Zagreb (HR)
(74) Representative: von Füner, Alexander, Dr.

(56) References cited:
- EP-A- 0 101 186
- EP-A- 0 137 132
- GB-A- 1 100 504
- JOURNAL OF CHEMICAL RESEARCH, 1988 pages 1239 - 1261 S.DJOKIC ET AL. 'Erythromycin Series. Part13. Synthesis and Structure Elucidation of 10-Dihydro-10-deoxo-11-meth yl-11-azaerythromycin A.'

## Description

The present invention relates to a new process for the preparation of azithromycin dihydrochloride, which is a pharmaceutically acceptable salt of the antibiotic azithromycin useful as an antibacterial agent with a broad-spectrum action.

### Prior Art

Azithromycin, a semi-synthetic macrolide antibiotic, a representative of azalide class (Kobrehel G. et al, BE 892,357, 7/1982; Bright G. M., US 4,474,768, 10/1984) possesses a broad spectrum of action against microbes including gram-negative bacteria and intracellular microorganisms. Its dihydrate form (Sumamed®) is used in pharmaceutical preparations suitable for peroral application for treating infectious bacterial diseases.

The same biological properties are known for its addition salt, azithromycin dihydrochloride, which owing to its good solubility in an aqueous medium can be used for parenteral application in pharmaceutically suitable forms (injections, infusions).

So far, two processes for the preparation of azithromycin dihydrochloride have been described in the literature. According to Bright (above-mentioned US patent) a reaction of azithromycin and pyridine hydrochloride is performed in methylene chloride and, after the evaporation of the solvent, azithromycin dihydrochloride is isolated from an aqueous solution by lyophilisation in a 54.4% yield.

According to Djoki 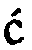 et al, J. Chem. Resarch (M), 1988, 1239-1261, azithromycin dihydrochloride is obtained by lyophilization of a hydrochloric aqueous solution of azithromycin (pH 6.4-6.5) in a 91.6% yield.

We have found - and this represents the object of the invention - that azithromycin dihydrochloride can be prepared by a new precipitation process, which is more acceptable from an economical and technical point of view than the processes hitherto described in the literature.

### Technical Solution

It has been found that azithromycin dihydrochloride can be obtained by the reaction of azithromycin mono- or dihydrate dissolved in lower (C₁-C₄) alcohols or lower (C₃-C₆) ketones with 2 equiv. of hydrogen chloride gas dissolved in a dry lower (C₁-C₄) alcohol at a concentration of 12-20% (w/v) and at a temperature of 10-15 °C, whereupon the product is precipitated by the addition of a non-solvent to the reaction mixture or by pouring the reaction mixture into a non-solvent at a temperature of 10-25 °C.

By the term "lower alcohols", alcohols such as methanol, ethanol, n-propanol, i-propanol, n-butanol and isomeric forms thereof are meant.

By the term "lower ketones", ketones such as dimethyl ketone, methylethyl ketone, isobutylmethyl ketone or similar compounds are meant, wherein azithromycin monoand dihydrate are well soluble and which also are miscible with non-solvents for the product such as ethers, preferably diisopropyl ether.

The solvent to non-solvent ratio may vary from 1:1.8 to 1:8, the most suitable ratio being 1:5.8 (v/v).

After the dropwise addition of the reactants has been finished, the reaction suspension is stirred for another hour at the same temperature interval, whereafter the obtained azithromycin dihydrochloride is filtered, washed with the cold non-solvent and dried in vacuum.

The process for the preparation of azithromycin dihydrochloride of the present invention is illustrated but in no way limited by the following examples.

### Example 1

To an azithromycin dihydrate solution (5 g, 0.0064 moles) in isopropanol (20 ml), 2.5 ml of a 18% hydrogen chloride gas solution in dry isopropanol (0.0124 moles of hydrogen chloride gas) were added dropwise under stirring within 5 minutes at a temperature of 10-15 °C. The resulting reaction mixture was added dropwise to diisopropyl ether (130 ml) under stirring within 30 minutes. The stirring of the reaction mixture was continued for another hour at room temperature, subsequently the precipitate was filtered, washed with cold isopropanol (5 ml) and dried for 5 hours in a vacuum dryer at 40 °C. 5.15 g (98.4%) of azithromycin dihydrochloride, m.p. 186-192 °C, were obtained.
- ¹H NMR (CD₃OD) δ ppm: 2.84 (s, 9H N(CH₃)₂ and NCH₃)
3.36 (s, 3H, OCH₃)
Analysis for C₃₈ H₇₂O₁₂N₂.2HCl
calc.: 8.63% Cl
found: 8.40% Cl

### Example 2

To an azithromycin dihydrate solution (5g, 0.0064 moles) in acetone (10 ml), 2.5 ml of a 18% hydrogen chloride gas solution in dry isopropanol (0.0124 moles of hydrogen chloride gas) were added dropwise under stirring within 5 minutes at a temperature of 10-15 °C. Subsequently, while keeping the same temperature, diisopropyl ether (60 ml) was added dropwise into the reaction mixture within 1 hour. After stirring for 1 hour at the same temperature, the precipitated salt was filtered off. 5.20 g (98.9%) of azithromycin dihydrochloride were obtained.

### Example 3

According to the process described in Example 2, from azithromycin dihydrate (2g, 0.0025 moles) dissolved in acetone (4 ml) by the reaction with 1.15 ml of a 12.9% hydrogen chloride gas solution in dry methanol (0.0041 moles of hydrogen chloride gas), 2.06 g (98.5%) of azithromycin dihydrochloride were obtained.

### Example 4

According to the process described in Example 1, from azithromycin monohydrate (2g, 0.0026 moles) dissolved in methanol (8 ml), 1.16 ml of 12.9% hydrogen chloride gas solution in dry methanol (0.0041 moles of hydrogen chloride gas) and diisopropyl ether (16 ml), 2.10 g (98.5%) azithromycin dihydrochloride were obtained.

## Claims

1. Process for the preparation of azithromycin dihydrochloride, characterized in that azithromycin monohydrate or azithromycin dihydrate dissolved in a lower (C₁-C₄) alcohol or lower (C₃-C₆) ketone is reacted with 1.6 to 2 equiv. of hydrogen chloride gas dissolved in a dry lower (C₁-C₄) alcohol at a concentration of 12-20% (w/v) and at a temperature from 10 °C to 15 °C, the obtained product is precipitated with a non-solvent at a temperature from 10 °C to 25 °C, the ratio solvent:non-solvent being from 1:1.8 to 1:8 (v/v), and then isolated by filtration.

2. Process according to claim 1, characterized in that the non-solvent is ether, particularly diethyl ether or diisopropyl ether.

## Patentansprüche

1. Verfahren zur Herstellung von Azithromycin-dihydrochloriden, dadurch gekennzeichnet, dass das in einem niedrigen (C₁-C₄)-Alkohol oder niedrigen (C₃-C₆)-Keton gelöste Azithromycin-monohydrat oder Azithromycin-dihydrat mit 1,6 bis 2 Äquivalenten eines in einem trockenen niedrigen (C₁-C₄)-Alkohol in einer Konzentration von 12-20% (Gew./Vol.) gelösten Chlorwasserstoffgases bei einer Temperatur von 10°C bis 15°C umgesetzt wird, das erhaltene Produkt mit einem Nichtlösungsmittel bei einer Temperatur von 10°C bis 25°C und einem Verhältnis von Lösungsmittel:Nichtlösungsmittel von 1:1,8 bis 1:8 (Vol./Vol.) gefällt und anschliessend durch Filtration isoliert wird.

2. Verfahren gemäss Anspruch 1, dadurch gekennzeichnet, dass das Nichtlösungsmittel ein Ether, besonders ein Diethylether oder Diisopropylether ist.

## Revendications

1. Procédé de préparation du dichlorhydrate d'azithromycine, caractérisé en ce que l'on fait réagir l'azithromycine monohydratée, ou l'azothromycine dihydratée, dissoute dans un alcool inférieur (C₁-C₄), ou une cétone inférieure (C₃-C₆) avec de 1,6 à 2 équivalents de chlorure d'hydrogène gazeux, dissous dans un alcool inférieur (C₁-C₄) sec, en une concentration de 12 à 20% (p/v) et à une température de 10°C à 15°C, on précipite le produit obtenu avec un non solvant, à une température de 10°C à 25°C, le rapport solvant:non solvant variant de 1:1,8 à 1:8 (v/v) et l'isole ensuite par filtration.

2. Procédé suivant la revendication 1, caractérisé en ce que le non solvant est l'éther, en particulier, l'éther diéthylique ou l'éther diisopropylique.
